(19) Europäisches Patentamt
European Patent Office
Office européen des brevets

(11) Veröffentlichungsnummer: **0 168 344**
**B1**

(12) # EUROPÄISCHE PATENTSCHRIFT

(45) Veröffentlichungstag der Patentschrift: **10.08.88**

(21) Anmeldenummer: **85810271.8**

(22) Anmeldetag: **10.06.85**

(51) Int. Cl.⁴: **C 07 C 43/225**, C 07 C 143/78, C 07 C 93/14, C 07 C 149/34, C 07 C 41/22

(54) **Verfahren zur Herstellung von alpha,alpha-Difluoralkyl-phenyläther-Derivaten.**

(30) Priorität: **15.06.84 CH 2918/84**
**15.06.84 CH 2917/84**
**20.02.85 CH 790/85**

(43) Veröffentlichungstag der Anmeldung: **15.01.86 Patentblatt 86/3**

(45) Bekanntmachung des Hinweises auf die Patenterteilung: **10.08.88 Patentblatt 88/32**

(84) Benannte Vertragsstaaten: **AT BE CH DE FR GB IT LI NL**

(56) Entgegenhaltungen:
**EP-A-0 084 743**
**EP-A-0 124 002**
**US-A-2 639 299**
**US-A-2 803 665**
**US-A-3 666 864**

**THE JOURNAL OF ORGANIC CHEMISTRY, Band 44, Nr. 16, 3. August 1979, Seiten 2907-2910, American Chemical Society; A.E. FEIRING: "Chemistry in hydrogen fluoride. 7. A novel synthesis of aryl trifluoromethyl ethers"**

(73) Patentinhaber: **CIBA- GEIGY AG, Klybeckstrasse 141, CH- 4002 Basel (CH)**

(72) Erfinder: **Siegrist, Urs, Rüttenenweg 61, CH- 4313 Möhlin (CH)**
Erfinder: **Indermühle, Jean, Dr., Thiersteinerallee 19, CH- 4053 Basel (CH)**
Erfinder: **Baumeister, Peter, St. Annaweg 24, CH- 4113 Flüh (CH)**

**EP 0 168 344 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein neues Verfahren zur Herstellung von α,α-Difluoralkyl-phenyläther-Derivaten und α-Chlor-α-fluoralkyl-phenyläther-Derivaten sowie neue α-Chlor-α-fluoralkylphenyläther-Derivate.

Die nach dem erfindungsgemässen neuen Verfahren herstellbaren α,α-Difluoralkyl-phenyläther-Derivate und α-Chlor-α-fluoralkylphenyläther-Derivate sind wertvolle Zwischenprodukte für herbizide und pflanzenwuchsregulierende Wirkstoffe. Solche Wirkstoffe sind mit ihren biologischen Wirkungen beispielsweise in den Europäischen Patentanmeldungspublikationen EP-A-23 422 und 44 808 beschrieben. Weiterhin bilden die α-Chlor-α-fluoralkylphenyläther Zwischenprodukte für α,α-Difluoralkylphenyl-äther-Derivate die ihrerseits ebenfalls Ausgangsstoffe für herbizide Sulfonylharnstoffe sind.

Die Erzeugung von Verbindungen mit α,α-Difluoralkyläther-Struktur ist schon verschiedentlich beschrieben worden: Organic Reactions, Vol. 21 (1974), Wiley; 1 - 124; J. Fluorine Chem. 24 (1984) 191 - 203; US-PS-2 803 665; oder Europäische Patentanmeldungspublikation EP-A-84 743. Die angewendeten Verfahren erweisen sich für den großtechnischen Einsatz als wenig geeignet, da einerseits teuere Ausgangschemikalien verwendet werden, und andererseits nur Thioäther erzeugt werden können, oder einerseits schwierig handhabbare Substanzen unter großem technischen Aufwand zum Einsatz kommen und andererseits die Reaktionsprodukte eine teilweise unbefriedigende Reinheit aufweisen.

Es besteht daher ein Bedürfnis nach einem breit anwendbaren Syntheseverfahren zur Herstellung von α,α-Difluoralkyl-phenyläther-Derivaten, das unter Verwendung billiger Ausgangsstoffe und Vermeidung eines großen technischen Aufwandes einheitliche Produkte in hohen Ausbeuten liefert.

Auch die Herstellung von Verbindungen mit α-Chlor-α-fluoralkyläther-Struktur ist schon beschrieben worden: Zh. Obshch. Khim. 1969, 39(4), 765 - 762 [C.A. 71 (1969) 60927h] oder Zh. Obshch. Khim. 1969, 38(7), 1503 - 1509 [C.A. 70 (1969) 3436c]. Die angewendeten Verfahren erweisen sich ebenfalls für den großtechnischen Einsatz als wenig geeignet, da einerseits nur Produktgemische unterschiedlicher isomerer Struktur hergestellt werden können und andererseits nur Thioäther erzeugt werden können.

Es besteht daher auch hier ein Bedürfnis nach einem breit anwendbaren Syntheseverfahren zur Herstellung von α-Chlor-α-fluoralkyl-phenyläther-Derivaten, das unter Verwendung billiger Ausgangsstoffe und Vermeidung eines großen technischen Aufwandes einheitliche Produkte in hohen Ausbeuten liefert.

Überraschenderweise wurde nun festgestellt, daß das erfindungsgemäße neue Verfahren zur Herstellung von α,α-Difluoralkyl-phenyläther-Derivaten die Anforderungen des bestehenden Bedürfnisses weitgehend befriedigt und gleichzeitig durch geringfügige Abwandlung der Reaktionsbedingungen zur Herstellung von α-Chlor-α-fluoralkyl-phenyläther-Derivaten geeignet ist und auch hier die bestehenden Anforderungen an ein für den großtechnischen Maßstab geeignetes Verfahren erfüllt.

Das erfindungsgemäße Verfahren zur Herstellung eines α,α-Difluoralkyl-phenyläther-Derivates der Formel I

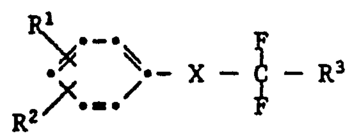 (I)

worin

$R^1$ Wasserstoff, Halogen, Amino, Nitro, $-SO_2-R^4$, $-S-R^5$, $-SO-R^6$ oder $-S-S-R^7$,

$R^2$ Wasserstoff, Halogen, Nitro, Hydroxyl oder $-SO_2-R^8$,

$R^3$ $C_1$-$C_5$-Halogenalkyl,

X Sauerstoff, Schwefel, -SO- oder $-SO_2$,

$R^4$ Hydroxyl, Halogen, Amino, -N=C=O, -NH-CO-Cl, -NH-CO-Br, $-NR^9R^{10}$, Benzyl, Phenyl, $C_1$-$C_4$-Alkyl oder $-NH-CO-NR^{11}R^{12}$,

$R^5$ und $R^6$ $C_1$-$C_4$-Alkyl, Phenyl oder Benzyl,

$R^7$ $C_1$-$C_4$-Alkyl, Phenyl oder Benzyl,

$R^8$ Hydroxyl oder Halogen,

$R^9$ und $R^{10}$ unabhängig voneinander $C_1$-$C_4$-Alkyl oder Benzyl und

$R^{11}$ und $R^{12}$ unabhängig voneinander Wasserstoff, $C_1$-$C_4$-Alkyl, Phenyl oder einen aromatischen Heterocyclus bedeuten,

ist dadurch gekennzeichnet, daß man eine Verbindung der Formel II

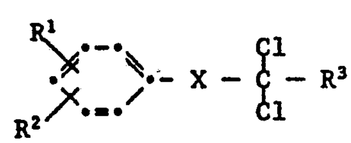 (II)

worin $R^1$, $R^2$, $R^3$ und X die unter Formel I gegebenen Bedeutungen haben, mit Fluorwasserstoff in Gegenwart einer katalytischen Menge eines Antimon(V)halogenids oder eines Antimontrichlorid/Halogengemisches fluoriert.

In den unter Formel I angegebenen Definitionen steht Halogen als Substituent für Fluor, Chlor, Brom oder

Iod, vorzugsweise für Fluor oder Chlor. Die gleiche Bedeutung und Bevorzugung hat Halogen als Teil von Halogenalkyl.

Alkyl steht beispielsweise für Methyl, Äthyl, i-Propyl, n-Propyl, die vier isomeren, Butyl- sowie die isomeren Pentylreste.

Halogenalkyl bedeutet sinngemäß im allgemeinen vorzugsweise Chlormethyl, Fluormethyl, Chloräthyl, Fluoräthyl, Dichlormethyl, Difluormethyl, Trichlormethyl, Trifluormethyl, Trichloräthyl, Trifluoräthyl, Tetrachloräthyl, Tetrafluoräthyl, Perchloräthyl, Perfluoräthyl, Perfluorpropyl, Perfluorbutyl oder Perfluorpentyl.

Aromatische Heterocyclen im Rahmen vorliegender Erfindung sind unsubstituierte oder substituierte Triazol-, Triazin- oder Pyrimidinringe. Besonders hervorzuheben sind 1,2,4-Triazol-3-yl-, 1,3,5-Triazin-2-yl- und 2-Pyrimidinylringsysteme, die vorzugsweise zweifach mit niederen Alkyl-, Alkoxy-, Halogenalkyl- oder Halogenalkoxyresten substituiert sind.

Die Reaktionsprodukte der Formel I sind entweder selbst herbizide Sulfonylharnstoffe oder sie können durch einen oder mehrere an sich bekannte Reaktionsschritte in herbizide Sulfonylharnstoffe überführt werden. So stellen Verbindungen der Formel I, in denen $R^1$ den Rest $-SO_2-NH-CO-NR^{11}R^{12}$ bedeutet, für den Fall, dass $R^{11}$ oder $R^{12}$ für einen Pyrimidin- oder Triazinring stehen, selbst herbizide Sulfonylharnstoffe dar. Steht $R^1$ für ein anderes Sulfonsäure-Derivat, etwa die freie Säure, ein Säureamid, ein Säurehalogenid, einen Isocyanatosulfonyl- oder einen Carbamoylsulfonylrest, so können diese Derivate durch an sich bekannte Reaktionsweisen in Sulfonylharnstoff-Derivate überführt werden. Ebenso sind dem Fachmann verschiedene Methoden bekannt, die es ihm erlauben, Verbindungen der Formel I, in denen $R^1$ für Wasserstoff, Halogen, Amino, Nitro, $-S-R^5$, $-SO-R^6$ oder $-S-S-R^7$ steht, in Phenylsulfonsäure-Derivate und daran anschliessend in herbizide Sulfonylharnstoffe zu überführen.

Zur Durchführung des erfindungsgemäßen Verfahrens (II → I) verwendet man als Fluorierungskatalysator ein Antimonhalogenid, in der das Antimonatom in der Oxidationsstufe V vorliegt. Der Einsatz dieses Katalysators ermöglicht gegenüber bekannten Verfahren gleichzeitig die Verwendung des relativ leicht zugänglichen Fluorwasserstoffs als Fluorierungsmittel und eine Senkung der Reaktionstemperatur. Durch die Absenkung der Reaktionstemperatur und die damit verbundene Druckminderung werden die betriebswirtschaftlichen Kosten für den Reaktor gesenkt. In günstigen Fällen kann die Reaktion bei Normaldruck oder leichtem Unterdruck ausgeführt werden. Zusätzlich erhöht sich gegenüber den bekannten Verfahren die Ausbeute an α,α-difluoriertem Produkt der Formel I.

Als Fluorierungskatalysator wird ein Antimon(V)halogenid verwendet. Wegen der einfachen Verfügbarkeit ist insbesondere Antimonpentachlorid zu erwähnen. Geeignet sind auch Katalysatorsysteme, die intermediär Antimon(V)halogenide bilden. Solche Katalysatorsysteme sind erfindungsgemäß Antimontrichlorid/Halogen-Gemische. Spezifisch zu nennen ist hier das Gemisch von Antimontrichlorid und Brom.

Der Katalysatoranteil im Reaktionsgemisch ist in weiten Grenzen variabel. So läßt sich die erfindungsgemäße Umsetzung mit 0,1 bis 50 Mol-Prozent an Antimon(V)halogenid, bezogen auf die eingesetzte Menge der Ausgangsverbindung der Formel II, durchführen. Für die technische Anwendung in einem Großreaktor brauchbare Reaktionsgeschwindigkeiten werden erzielt, wenn man als Katalysator zwischen 1 und 20 Mol-Prozent, insbesondere zwischen 5 und 20 Mol-Prozent, Antimon(V)halogenid verwendet.

Durch die Verwendung des Fluorierungskatalysators ist es möglich, die Reaktion bei milden Reaktionsbedingungen, d.h. bei relativ niedriger Reaktionstemperatur durchzuführen. So liegt die Reaktionstemperatur im allgemeinen zwischen -20°C und +100°C. Vorzugsweise hält man die Reaktionstemperatur zwischen -10°C und +20°C.

Der als Fluorierungsmittel eingesetzte Fluorwasserstoff wird mindestens in äquivalenter Menge eingesetzt. Im allgemeinen wird ein Überschuss an Fluorwasserstoff von 0,5 bis 2 Mol verwendet. Die Reaktion kann ohne Lösungsmittel oder in Gegenwart eines inerten Lösungsmittels ausgeführt werden. Geeignete inerte Lösungsmittel sind: Amide wie Dimethylformamid oder N-Methylpyrrolidon; aromatische Kohlenwasserstoffe wie Benzol, Toluol oder Xylol; Sulfoxide wie Dimethylsulfoxid; Sulfone wie Sulfolan oder Halogenkohlenwasserstoffe wie Methylenchlorid, Chloroform, Tetrachlorkohlenstoff, Trichloräthan, Chlorbenzol, Trichlorfluormethan, Dichlordifluormethan, 1,2-Dichlor-1,1,2,2-tetrafluoräthan, Chlortrifluormethan oder Fluorwasserstoff. Falls der Fluorwasserstoff selbst als Lösungsmittel eingesetzt wird, kann der Überschuß an Fluorwasserstoff aber auch sehr viel grösser sein.

Das erfindungsgemäße Verfahren wird vorzugsweise bei Normaldruck durchgeführt. Jedoch kann es sich in einzelnen Fällen als notwendig erweisen, die Reaktion bei Unter- oder Überdruck ablaufen zu lassen. Die Reaktionsdrücke können jedoch im allgemeinen zwischen 0,1 bar und 20 bar gehalten werden.

Zur vollständigen Umsetzung des Ausgangsmaterials der Formel II in die Verbindung der Formel I nach dem erfindungsgemäßen Verfahren werden im allgemeinen Reaktionszeiten zwischen einigen Minuten und 24 Stunden benötigt. Vorzugsweise wählt man die Reaktionsbedingungen so, daß die Reaktionszeiten zwischen 0,5 und 4 Stunden liegen.

Ein erfindungsgemäß bevorzugtes Verfahren zur Herstellung von α,α-Difluoralkyl-phenyläther der Formel I ist dadurch gekennzeichnet, daß man die Umsetzung in Gegenwart von 0,1 bis 50 Mol-Prozent an Antimon(V)halogenid bei einer Temperatur zwischen -20°C und +100°C unter einem Druck zwischen 0,1 bar und 20 bar ausführt.

Eine besonders bevorzugte Ausführungsform ist dadurch gekennzeichnet, daß man die Umsetzung in Gegenwart von 1 bis 20 Mol-Prozent an Antimon(V)chlorid bei einer Temperatur zwischen -10°C und +20°C unter Normaldruck in flüssigem Fluorwasserstoff ausführt.

Das erfindungsgemäße Verfahren (II → I) ist in besonderer Weise dazu geeignet, α,α-Difluoralkyl-phenyläther der engeren Formel Ia

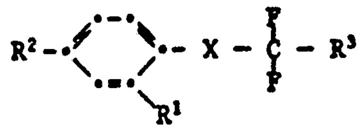 (Ia)

herzustellen, worin

$R^1$ Wasserstoff, Halogen, Nitro, Amino, $-SO_2-R^4$, $-S-R^5$ oder $-SO-R^6$,

$R^2$ Wasserstoff, Halogen oder Nitro,

$R^3$ $C_1$-$C_3$-Perfluoralkyl,

X Sauerstoff oder Schwefel,

$R^4$ Hydroxyl, Amino, Phenyl, Benzyl, $C_1$-$C_4$-Alkyl oder Halogen, vorzugsweise Fluor oder Chlor und

$R^5$ und $R^6$ $C_1$-$C_4$-Alkyl oder Benzyl bedeuten.

Von diesen Produkten der Formel I werden bevorzugt diejenigen nach dem neuen erfindungsgemäßen Verfahren hergestellt, in denen

$R^1$ für $-SO_2NH_2$, Wasserstoff oder Chlor,

$R^2$ für Wasserstoff oder Chlor,

$R^3$ für $C_1$-$C_3$-Perfluoralkyl und

X für Sauerstoff oder Schwefel stehen.

Von diesen Verbindungen sind diejenigen hervorzuheben, in denen eines von $R^1$ und $R^2$ für Chlor und das andere für Wasserstoff oder Chlor steht. Als vorzugsweise hervorzustellende Einzelverbindungen der Formel I sind neben 2-Perfluoräthoxyphenylsulfonamid vor allem zu nennen: 2,4-Dichlor-perfluoräthoxybenzol, 2-Perfluoräthoxy-chlorbenzol und 4-Perfluoräthoxy-chlorbenzol.

Diese besonders bevorzugten Verbindungen der Formel I, ausgewählt aus der Gruppe 2-Perfluoräthoxyphenylsulfonamid, 2,4-Dichlor-perfluoräthoxybenzol, 2-Perfluoräthoxychlorbenzol oder 4-Perfluoräthoxychlorbenzol, werden vorzugsweise hergestellt, indem man 2-(1,1-Dichlor-2,2,2-trifluoräthoxy)-phenylsulfonamid, 1-(1,1-Dichlor-2,2,2-trifluoräthoxy)-2,4-dichlorbenzol, 2-(1,1-Dichlor-2,2,2-trifluoräthoxy)-chlorbenzol oder 4-(1,1-Dichlor-2,2,2-trifluoräthoxy)-chlorbenzol in Gegenwart von 5 bis 20 Mol-Prozent Antimon(V)chlorid bei einer Temperatur zwischen -10°C und +10°C unter Normaldruck in flüssigem Fluorwasserstoff fluoriert.

In einer Variante kann das erfindungsgemäße Verfahren (II - I) auch zweistufig ausgeführt werden, indem man zunächst die Verbindung der Formel II in Gegenwart von 0,1 bis 5 Mol-Prozent Antimon(V)halogenid mit Fluorwasserstoff in das Zwischenprodukt der Formel III

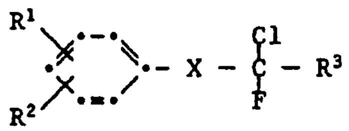 (III)

worin $R^1$, $R^2$, $R^3$ und X die unter Formel I im Anspruch 1 gegebenen Bedeutungen haben, überführt und dieses in Gegenwart von 1 bis 20 Mol-Prozent an Antimon(V)halogenid mit Fluorwasserstoff zur Verbindung der Formel I umsetzt, wobei die Umsetzung der Verbindung der Formel II zur Verbindung der Formel III bei einer Temperatur zwischen -20°C und 0°C und die weitere Umsetzung des Zwischenprodukts der Formel III bei einer Temperatur zwischen 0°C und +100°C durchgeführt wird.

Die Zwischenprodukte der Formel III können dabei wahlweise isoliert werden oder ohne Isolierung direkt zur Weiterreaktion in der zweiten Stufe eingesetzt werden. Die Reaktionsbedingungen für die zweistufige Variante wie Druck, Temperatur, Lösungsmittel und Katalysatorkonzentration werden mit den aufgezeigten Einschränkungen im Rahmen der Bedingungen des erfindungsgemäßen einstufigen Verfahrens gewählt.

Wird aus betriebstechnischen Gründen eine zweistufige Verfahrensführung gewünscht, ist es notwendig, die erste Stufe mit niedriger Katalysatorkonzentration und niedriger Temperatur zu steuern. Um die zweite Stufe ablaufen zu lassen, werden zweckmäßigerweise die Katalysatorkonzentration und die Temperatur erhöht. Die Katalysatorkonzentrationen und Reaktionstemperaturen für die beiden Reaktionsstufen können je nach Konstitution und Substitution des Phenylkerns der Ausgangsverbindung der Formel II unterschiedlich sein und müssen der jeweiligen Reaktivität des Ausgangs- und Zwischenproduktmoleküls angepasst werden.

Die Ausführung des erfindungsgemäßen Fluorierungsverfahrens kann gemäß üblicher chemischer Verfahrenstechnik in diskontinuierlich oder kontinuierlich arbeitenden Reaktoren erfolgen. Je nach Reaktionstemperatur und -druck ist das Reaktionsmedium dabei flüssig oder gasförmig.

Die Ausgangsverbindungen der Formel II sind bekannt oder sie können nach bekannten Methoden hergestellt werden. Vorzugsweise erhält man die α,α-Dichloralkyl-phenylätherderivate der Formel II aus entsprechenden Alkancarbonsäurephenylestern durch Behandlung mit üblichen Chlorierungsmitteln wie Phosphorpentachlorid, Phosphortrichlorid, Chlor, Phosphoroxychlorid oder Thionylchlorid. Ebenso kann man die Verbindungen der Formel II durch α-Chlorierung von entsprechenden Phenylalkyläthern mit Chlorgas herstellen.

Die erste Stufe des oben genannten Verfahrens zur Herstellung der Verbindungen der Formel I dient gleichzeitig als unabhängiges Verfahren zur Herstellung von α-Chlor-α-fluoralkyl-phenyläther-Derivaten der Formel III.

Dieses Verfahren zur Herstellung eines α-Chlor-α-fluoralkyl-phenyläther-Derivates der Formel III

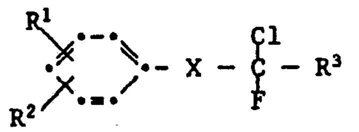 (III)

worin

$R^1$ Wasserstoff, Halogen, Amino, Nitro, $-SO_2-R^4$, $-S-R^5$, $-SO-R^6$ oder $-S-S-R^7$,

$R^2$ Wasserstoff, Halogen, Nitro, Hydroxyl oder $-SO_2-R^8$,

$R^3$ $C_1$-$C_5$-Halogenalkyl,

X Sauerstoff, Schwefel, -SO- oder $-SO_2-$,

$R^4$ Hydroxyl, Halogen, Amino, Benzyl, Phenyl, $C_1$-$C_4$-Alkyl, -N=C=O, -NH-CO-Cl, -NH-CO-Br, $-NR^9R^{10}$ oder $-NH-CO-NR^{11}R^{12}$,

$R^5$ und $R^6$ $C_1$-$C_4$-Alkyl, Phenyl oder Benzyl,

$R^7$ $C_1$-$C_4$-Alkyl, Phenyl oder Benzyl,

$R^8$ Hydroxyl oder Halogen,

$R^9$ und $R^{10}$ unabhängig voneinander $C_1$-$C_4$-Alkyl oder Benzyl und

$R^{11}$ und $R^{12}$ unabhängig voneinander Wasserstoff, $C_1$-$C_4$-Alkyl, Phenyl oder einen aromatischen Heterocyclus bedeuten,

ist dadurch gekennzeichnet, daß man eine Verbindung der Formel II

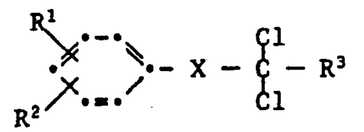 (II)

worin $R^1$, $R^2$, $R^3$ und X die unter Formel III gegebenen Bedeutungen haben, mit Fluorwasserstoff in Gegenwart einer katalytischen Menge eines Antimon(V)halogenids fluoriert.

Die unter der Formel III angegebenen Begriffe zur Definition der Einzelsubstituenten umfassen die gleichen individuellen Substituenten, welche bereits unter Formel I genannt sind.

Die Zwischenprodukte der Formel III sind entweder selbst herbizide Sulfonylharnstoffe oder sie können durch einen oder mehrere an sich bekannte Reaktionsschritte in herbizide Sulfonylharnstoffe überführt werden. So kann durch fortgesetzte Fluorierung das α-Chloratom der Verbindung der Formel III ebenfalls durch Fluor ersetzt werden, wodurch herbizide Sulfonylharnstoff-Derivate der Formel I entstehen. Ebenso stellen Verbindungen der Formel III, in denen $R^1$ für den Rest $-SO_2-NH-CO-NR^{11}R^{12}$ steht, für den Fall, dass $R^{11}$ oder $R^{12}$ einen Pyrimidin- oder Triazinring bedeutet, herbizide Sulfonylharnstoffe dar. Steht $R^1$ für ein anderes Sulfonsäure-Derivat, etwa die freie Säure, ein Säureamid, ein Säurehalogenid, einen Isocyanatosulfonyl- oder einen Carbamoylsulfonylrest, so können diese Derivate durch an sich bekannte Reaktionsweisen in Sulfonylharnstoff-Derivate überführt werden. Ebenso sind dem Fachmann verschiedene Methoden bekannt, die es ihm erlauben, Verbindungen der Formel III, in denen $R^1$ für Wasserstoff, Halogen, Amino, Nitro, $-S-R^5$, $-SO-R^6$ oder $-S-S-R^7$ steht, in Phenylsulfonsäure-Derivate und daran anschliessend in herbizide Sulfonylharnstoffe zu überführen.

Zur Durchführung des Verfahrens zur Herstellung der Verbindungen der Formel III verwendet man als Fluorierungskatalysator ein Antimonhalogenid, in der das Antimonatom in der Oxidationsstufe V vorliegt. Der Einsatz dieses Katalysators ermöglicht gleichzeitig die Verwendung des relativ leicht zugänglichen Fluorwasserstoffs als Fluorierungsmittel und eine Senkung der Reaktionstemperatur. Durch die Absenkung der Reaktionstemperatur und die damit verbundene Druckminderung werden die betriebswirtschaftlichen Kosten für den Reaktor gesenkt. In günstigen Fällen kann die Reaktion bei Normaldruck oder leichtem Unterdruck ausgeführt werden.

Zur Durchführung dieses Mono-fluorierungsverfahrens werden niedrigere Katalysatorkonzentrationen und/oder Reaktionstemperaturen angewendet als bei der Difluorierung des gleichen Ausgangsprodukts. Je nach der Reaktivität des Ausgangsmaterials der Formel II können die anzuwendenden Reaktionsparameter überlappen. So kann beispielsweise bei einer bestimmten Reaktionstemperatur und einer bestimmten Katalysatorkonzentration, eine reaktionsträge Verbindung der Formel II zur Verbindung der Formel III monofluoriert werden, während bei gleichen Reaktionsbedingungen eine reaktionsaktivere Verbindung zur Verbindung der Formel I difluoriert wird. Die geeigneten Reaktionsbedingungen werden jeweils im Hinblick auf das gewünschte Produkt der Formel II oder III speziell ausgewählt.

Vorzugsweise wird für die Reaktion (II → III) als Fluorierungskatalysator ein Antimon(V)halogenid verwendet. Wegen der einfachen Verfügbarkeit ist insbesondere Antimonpentachlorid zu erwähnen.

Geeignet sind auch Katalysatorsysteme, die intermediär Antimon(V)halogenide bilden. Solche Katalysatorsysteme sind erfingungsgemäß Antimontrichlorid/Halogen-Gemische. Spezifisch zu nennen ist hier

das Gemisch von Antimontrichlorid und Brom.

Der Katalysatoranteil im Reaktionsgemisch ist bei der Umsetzung (II → III) in weiten Grenzen variabel. So läßt sich die Umsetzung mit 0,1 bis 50 Mol-Prozent an Antimon(V)halogenid, bezogen auf die eingesetzte Menge der Ausgangsverbindung der Formel II, durchführen. Für die technische Anwendung in einem Großreaktor brauchbare Reaktionsgeschwindigkeiten werden erzielt, wenn man als Katalysator zwischen 1 und 5 Mol-Prozent, Antimon(V)halogenid verwendet.

Durch die Verwendung des Fluorierungskatalysators ist es möglich die Reaktion (II → III) bei milden Reaktionsbedingungen, d.h. bei relativ niedriger Reaktionstemperatur durchzuführen. So liegt die Reaktionstemperatur im allgemeinen zwischen -20°C und +100°C. Vorzugsweise hält man die Reaktionstemperatur zwischen -10°C und +20°C. Der als Fluorierungsmittel eingesetzte Fluorwasserstoff wird mindestens in äquivalenter Menge eingesetzt. Im allgemeinen wird ein Überschuß an Fluorwasserstoff von 0,5 bis 2 Mol verwendet. Die Reaktion kann ohne Lösungsmittel oder in Gegenwart eines inerten Lösungsmittels ausgeführt werden. Geeignete inerte Lösungsmittel sind: Amide wie Dimethylformamid oder N-Methylpyrrolidon; aromatische Kohlenwasserstoffe wie Benzol, Toluol oder Xylol; Sulfoxide wie Dimethylsulfoxid; Sulfone wie Sulfolan oder Halogenkohlenwasserstoffe wie Methylenchlorid, Chloroform, Tetrachlorkohlenstoff, Trichloräthan, Chlorbenzol, Trichlorfluormethan, Dichlordifluormethan, 1,2-Dichlor-1,1,2,2-tetrafluoräthan, Chlortrifluormethan oder Fluorwasserstoff. Falls der Fluorwasserstoff selbst als Lösungsmittel eingesetzt wird, kann der Überschuß an Fluorwasserstoff aber auch sehr viel größer sein. Das Verfahren (II → III) wird vorzugsweise bei Normaldruck durchgeführt. Jedoch kann es sich in einzelnen Fällen als notwendig erweisen, die Reaktion bei Unter- oder Überdruck ablaufen zu lassen. Die Reaktionsdrücke können jedoch im allgemeinen zwischen 0,1 bar und 20 bar gehalten werden.

Zur vollständigen Umsetzung des Ausgangsmaterials der Formel II in das Produkt der Formel III werden im allgemeinen Reaktionszeiten zwischen einigen Minuten und 24 Stunden benötigt. Vorzugsweise wählt man die Reaktionsbedingungen so, daß die Reaktionszeiten zwischen 0,5 und 4 Stunden liegen.

Ein bevorzugtes Verfahren zur Herstellung von α-Chlor-α-fluoralkyl-phenyläther der Formel III ist dadurch gekennzeichnet, daß man die Umsetzung in Gegenwart von 0,1 bis 50 Mol-Prozent an Antimon(V)halogenid bei einer Temperatur zwischen -20°C und +100°C unter einem Druck zwischen 0,1 bar und 20 bar ausführt. Eine besonders bevorzugte Ausführungsform ist dadurch gekennzeichnet, daß man die Umsetzung in Gegenwart von 1 bis 20 Mol-Prozent an Antimon(V)chlorid bei einer Temperatur zwischen -10°C und +20°C unter Normaldruck in flüssigem Fluorwasserstoff ausführt.

Das erfindungsgemäße Verfahren ist in besonderer Weise dazu geeignet, α-Chlor-α-fluoralkyl-phenyläther der engeren Formel IIIa

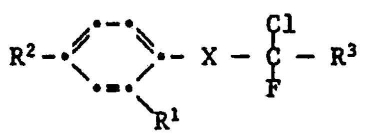 (IIIa)

herzustellen, worin

$R^1$ Wasserstoff Halogen, Nitro, Amino, $-SO_2-R^4$, $-S-R^5$, $-SO-R^6$,
$R^2$ Wasserstoff Halogen oder Nitro,
$R^3$ $C_1$-$C_3$-Perluoralkyl,
X Sauerstoff oder Schwefel,
$R^4$ Hydroxyl, Amino, Phenyl, Benzyl, $C_1$-$C_4$-Alkyl oder Halogen, vorzugsweise Fluor oder Chlor, und
$R^5$ und $R^6$ $C_1$-$C_4$-Alkyl oder Benzyl bedeuten.

Von diesen Zwischenprodukten werden bevorzugt diejenigen nach dem neuen erfindungsgemäßen Verfahren hergestellt, in denen $R^1$ für $-SO_2NH_2$, Wasserstoff oder Chlor, $R^2$ für Wasserstoff oder Chlor, $R^3$ für $C_1$-$C_3$-Perfluoralkyl und X für Sauerstoff oder Schwefel stehen. Von diesen Verbindungen sind diejenigen hervorzuheben, in denen eines von $R^1$ und $R^2$ für Chlor und das andere für Wasserstoff oder Chlor steht. Als vorzugsweise hervorzustellende Einzelverbindungen der Formel III sind neben 2-(1-Chlor-1,2,2,2-tetrafluoräthoxy)-phenyl-sulfonamid vor allem zu nennen: 2,4-Dichlor-(1-chlor-1,2,2,2-tetrafluoräthoxy)-benzol, 2-(1-Chlor-1,2,2,2-tetrafluoräthoxy)-chlorbenzol und 4-(1-Chlor-1,2,2,2-tetrafluoräthoxy)-chlorbenzol.

Diese besonders bevorzugten Verbindungen der Formel III, ausgewählt aus der Gruppe 2-(1-Chlor-1,2,2,2-tetrafluoräthoxy)-phenylsulfonamid, 2,4-Dichlor-(1-chlor-1,2,2,2-tetrafluoräthoxy)-benzol, 2-(1-Chlor-1,2,2,2-tetrafluoräthoxy)-chlorbenzol oder 4-(1-Chlor-1,2,2,2-tetrafluoräthoxy)-chlorbenzol werden vorzugsweise hergestellt, indem man 2-(1,1,-Dichlor-2,2,2-trifluoräthoxy)-phenylsulfonamid, 1-(1,1,-Dichlor-2,2,2-trifluoräthoxy)-2,4-dichlorbenzol, 2-(1,1,-Dichlor-2,2,2-trifluoräthoxy)-chlorbenzol oder 4-(1,1-Dichlor-2,2,2-trifluoräthoxy)-chlorbenzol in Gegenwart von 1 bis 5 Mol-Prozent Antimon(V)chlorid bei einer Temperatur zwischen -10°C und 0°C unter Normaldruck in flüssigem Fluorwasserstoff fluoriert.

Die Ausführung des Fluorierungsverfahrens (II → III) kann gemäß üblicher chemischer Verfahrenstechnik in diskontinuierlich oder kontinuierlich arbeitenden Reaktoren erfolgen. Je nach Reaktionstemperatur und -druck ist das Reaktionsmedium dabei flüssig oder gasförmig.

Einige der nach dem Verfahren hergestellten Zwischenprodukte der Formel III sind neu und stellen daher einen weiteren Gegenstand der vorliegenden Erfindung dar. Diese neuen Verbindungen entsprechen den Unterformeln IIIb

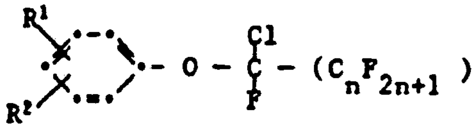 (IIIb),

worin $R^1$ und $R^2$ die unter Formel III gegebenen Bedeutungen haben und n für eine Zahl von eins bis fünf steht, und IIIc

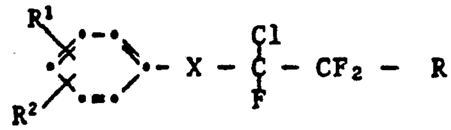 (IIIc),

worin $R^1$ und $R^2$ die im Anspruch 1 unter Formel III gegebenen Bedeutungen haben, X für Schwefel, SO oder $SO_2$ und R für Fluor oder $C_1$-$C_4$-Perhalogenalkyl stehen.

In den angeschlossenen Tabellen 1 bis 3 sind beispielhaft einige Ausgangs-, Zwischen- und Endprodukte der Formel I, II und III aufgelistet, die nach dem erfindungsgemäßen Verfahren umgesetzt oder erhalten werden können.

**Tabelle 1:**

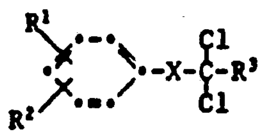

| Verb. Nr. | $R^1$ | $R^2$ | $R^3$ | X | Physikalische Daten |
|---|---|---|---|---|---|
| 1.1 | H | H | $CF_3$ | O | Sdp. 183°C |
| 1.2 | 4-Cl | H | $CF_3$ | O | Sdp. 218°C |
| 1.3 | 2-Cl | H | $CF_3$ | O | Sdp. 222°C |
| 1.4 | 4-Cl | 2-$NO_2$ | $CF_3$ | O | |
| 1.5 | 2-Cl | 4-Cl | $CF_3$ | O | |
| 1.6 | 2-$NH_2$ | H | $CF_3$ | O | |
| 1.7 | 2-$SO_2$-$NH_2$ | H | $CF_3$ | O | |
| 1.8 | 2-$SO_2$-OH | H | $CF_3$ | O | |
| 1.9 | 2-$SO_2$-Cl | H | $CF_3$ | O | |
| 1.10 | 2-$SO_2$-OH | H | $CF_3$ | O | |
| 1.11 | 2-$SO_2$-$NH_2$ | H | $CF_3$ | S | |
| 1.12 | 2-$SO_2$-$NH_2$ | H | $CCl_3$ | O | |
| 1.13 | 2-$SO_2$-Cl | H | $CCl_3$ | O | |
| 1.14 | 2-$NO_2$ | H | $CF_3$ | O | |
| 1.15 | 2-$NO_2$ | H | $CHF_2$ | O | |
| 1.16 | 2-$NH_2$ | H | $CHF_2$ | O | |
| 1.17 | 2-$NO_2$ | H | $CF_3$ | S | |
| 1.18 | 2-$NO_2$ | H | $C_3H_7$-n | O | |
| 1.19 | 2-$NH_2$ | H | $CF_3$ | S | |
| 1.20 | 4-Cl | 3-$NO_2$ | $CF_3$ | O | |
| 1.21 | 4-Cl | 3-Cl | $CF_3$ | O | |
| 1.22 | 4-Cl | 3-$NO_2$ | $CF_3$ | S | |
| 1.23 | 4-Cl | 2-$NO_2$ | $CF_3$ | S | |
| 1.24 | 2-Cl | H | $CF_3$ | S | |
| 1.25 | 2-S-S-$C_6H_5$ | H | $CF_3$ | O | |
| 1.26 | 2-S-$CH_2$-$C_6H_5$ | H | $CF_3$ | O | |
| 1.27 | H | H | $CF_3$ | S | Sdp. 218°C |
| 1.28 | 2-$SO_2$-Cl | 5-$SO_2$-Cl | $CF_3$ | O | |

**Tabelle 2:**

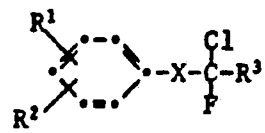

| Verb. Nr. | $R^1$ | $R^2$ | $R^3$ | X | Physikalische Daten |
|---|---|---|---|---|---|
| 2.1 | H | H | $CF_3$ | O | Sdp. 150°C |
| 2.2 | 4-Cl | H | $CF_3$ | O | Sdp. 181°C |
| 2.3 | 2-Cl | H | $CF_3$ | O | |
| 2.4 | 4-Cl | $2-NO_2$ | $CF_3$ | O | |
| 2.5 | 2-Cl | 4-Cl | $CF_3$ | O | |
| 2.6 | $2-NH_2$ | H | $CF_3$ | O | |
| 2.7 | $2-SO_2-NH_2$ | H | $CF_3$ | O | |
| 2.8 | $2-SO_2-OH$ | H | $CF_3$ | O | |
| 2.9 | $2-SO_2-Cl$ | H | $CF_3$ | O | |
| 2.10 | $2-SO_2-OH$ | H | $C_2F_5$ | O | |
| 2.11 | $2-SO_2-NH_2$ | H | $CF_3$ | S | |
| 2.12 | $2-SO_2-NH_2$ | H | $CCl_3$ | O | |
| 2.13 | $2-SO_2-Cl$ | H | $CCl_3$ | O | |
| 2.14 | $2-NO_2$ | H | $CF_3$ | O | |
| 2.15 | $2-NO_2$ | H | $CHF_2$ | O | |
| 2.16 | $2-NH_2$ | H | $CHF_2$ | O | |
| 2.17 | $2-NO_2$ | H | $CF_3$ | S | |
| 2.18 | $2-NO_2$ | H | $C_3H_7$-n | O | |
| 2.19 | $2-NH_2$ | H | $CF_3$ | S | |
| 2.20 | 4-Cl | $3-NO_2$ | $CF_3$ | O | |
| 2.21 | 4-Cl | 3-Cl | $CF_3$ | O | |
| 2.22 | 4-Cl | $3-NO_2$ | $CF_3$ | S | |
| 2.23 | 4-Cl | $2-NO_2$ | $CF_3$ | S | |
| 2.24 | 2-Cl | H | $CF_3$ | S | |
| 2.25 | $2-S-S-C_6H_5$ | H | $CF_3$ | O | |
| 2.26 | $2-S-CH_2-C_6H_5$ | H | $CF_3$ | O | |
| 2.27 | H | H | $CF_3$ | S | Sdp. 178 - 179°C |
| 2.28 | $2-SO_2-Cl$ | $5-SO_2-Cl$ | $CF_3$ | O | |

**Tabelle 3:**

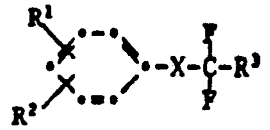

| Verb. Nr. | $R^1$ | $R^2$ | $R^3$ | X | Physikalische Daten |
|---|---|---|---|---|---|
| 3.1 | H | H | $CF_3$ | O | Sdp. 118° C |
| 3.2 | 4-Cl | H | $CF_3$ | O | Sdp. 160° C |
| 3.3 | 2-Cl | H | $CF_3$ | O | Sdp. 154° C |
| 3.4 | 4-Cl | $2-NO_2$ | $CF_3$ | O | |
| 3.5 | 2-Cl | 4-Cl | $CF_3$ | O | |
| 3.6 | $2-NH_2$ | H | $CF_3$ | O | Sdp. 175° C |
| 3.7 | $2-SO_2-NH_2$ | H | $CF_3$ | O | Smp. 148° C |
| 3.8 | $2-SO_2-OH$ | H | $CF_3$ | O | |
| 3.9 | $2-SO_2-Cl$ | H | $CF_3$ | O | |
| 3.10 | $2-SO_2-OH$ | H | $C_2F_5$ | O | |
| 3.11 | $2-SO_2-NH_2$ | H | $CF_3$ | S | |
| 3.12 | $2-SO_2-NH_2$ | H | $CCl_3$ | O | |
| 3.13 | $2-SO_2-Cl$ | H | $CCl_3$ | O | |
| 3.14 | $2-NO_2$ | H | $CF_3$ | O | |
| 3.15 | $2-NO_2$ | H | $CHF_2$ | O | |
| 3.16 | $2-NH_2$ | H | $CHF_2$ | O | |
| 3.17 | $2-NO_2$ | H | $CF_3$ | S | |
| 3.18 | $2-NO_2$ | H | $C_3H_7$-n | O | |
| 3.19 | $2-NH_2$ | H | $CF_3$ | S | |
| 3.20 | 4-Cl | $3-NO_2$ | $CF_3$ | O | |
| 3.21 | 4-Cl | 3-Cl | $CF_3$ | O | |
| 3.22 | 4-Cl | $3-NO_2$ | $CF_3$ | S | |
| 3.23 | 4-Cl | $2-NO_2$ | $CF_3$ | S | |
| 3.24 | 2-Cl | H | $CF_3$ | S | |
| 3.25 | $2-S-S-C_6H_5$ | H | $CF_3$ | O | |
| 3.26 | $2-S-CH_2-C_6H_5$ | H | $CF_3$ | O | |
| 3.27 | H | H | $CF_3$ | S | Sdp. 148° C |
| 3.28 | $2-SO_2-Cl$ | $5-SO_2-Cl$ | $CF_3$ | O | |

Die anschliessenden Beispiele dienen der näheren Illustration der Erfindung.

Beispiel 1: 4-Perfluoräthoxy-chlorbenzol.

In einem 300 ml Monel-Reaktor, der mit Rührwerk, Thermometer und Rückflusskühler ausgerüstet ist, werden 55,9 g (0,2 Mol) 4-(1,1-Dichlor-2,2,2-trifluoräthoxy)-chlorbenzol und 6,0 g (0,02 Mol; entsprechend 10 Mol-%) Antimonpentachlorid vorgelegt. Bei einer Temperatur zwischen -10°C und 0°C lässt man 100 g Fluorwasserstoff zulaufen. Der entstehende Chlorwasserstoff wird über den Rückflusskühler aus der Apparatur abgeleitet. Nach 2,5 Stunden werden der Reaktionsmischung nochmals 6,0 g (0,02 Mol) Antimonpentachlorid zugesetzt. Nach insgesamt 5 Stunden Reaktionsdauer wird der Fluorwasserstoff abdestilliert, der Rückstand in 250 ml Methylenchlorid gelöst und mit 50 g Kaliumfluorid versetzt. Durch destillative Trennung der Lösung erhält man 45,1 g (91,5 % d.Th.) 4-Perfluoräthoxy-chlorbenzol, Sdp. 151° C, $n_D^{25} = 1,4080$.

Beispiel 2: 4-Perfluoräthoxy-chlorbenzol.

In einem 500 ml Reaktor aus Polytetrafluoräthylen, der mit Rührwerk, Thermometer und Rückflusskühler ausgerüstet ist, werden 55,9 g (0,2 Mol) 4-(1,1-Dichlor-2,2,2-trifluoräthoxy)-chlorbenzol in 100 ml Fluorwasserstoff vorgelegt. Bei einer Temperatur von +10°C werden 3,0 g (0,01 Mol; entsprechend 5 Mol-%) Antimonpentachlorid zugegeben. Der entstehende Chlorwasserstoff wird über den Rückflusskühler aus dem Reaktor entfernt. Nach 2 Stunden bei +10°C lässt die Chlorwasserstoffentwicklung nach. Der überschüssige Fluorwasserstoff wird abdestilliert, der Rückstand in 200 ml Methylenchlorid gelöst und mit Wasser extrahiert. Durch destillative Trennung der organischen Phase erhält man 44,9 g (90,8 % d.Th.) 4-Perfluoräthoxy -chlorbenzol, Sdp. 151° C.

Beispiel 3: Perfluoräthoxy-benzol.

In einem 500 ml Polytetrafluoräthylen-Reaktor, der mit Rühwerk, Thermometer und Rückflusskühler ausgerüstet ist, werden 49,0 g (0,2 Mol) 1,1-Dichlor-2,2,2-trifluoräthoxybenzol in 100 ml Fluorwasserstoff vorgelegt. Bei einer Temperatur zwischen -5°C und 0°C werden 7,0 g (entsprechend 12 Mol-%) Antimonpentachlorid zugegeben. Der entstehende Chlorwasserstoff wird über den Rückflusskühler aus dem Reaktor entfernt. Nach 5 Stunden bei 0°C lässt die Chlorwasserstoffentwicklung nach. Der überschüssige

Fluorwasserstoff wird abdestilliert, der Rückstand in 250 ml Methylenchlorid gelöst und mit Wasser extrahiert. Durch destillative Trennung der organischen Phase erhält man 27,4 g (64,5 % d.Th.) Perfluroäthoxybenzol, Sdp. 118° C, $n_D^{25}$ = 1,3790.

Beispiel 4: 2-Perfluoräthoxy-chlorbenzol.

In einem 2 l Polytetrafluoräthylen-Reaktor, der mit Rührwerk, Thermometer und Rückflusskühler ausgerüstet ist, werden 279,5 g (1 Mol) 2-(1,1-Dichlor-2,2,2-trifluoräthoxy)-chlorbenzol in 500 ml Fluorwasserstoff vorgelegt. Bei einer Temperatur zwischen -5° C und 0° C werden 70,0 g (entsprechend 25 Mol-%) Antimonpentachlorid zugegeben. Der entstehende Chlorwasserstoff wird über den Rückflusskühler aus dem Reaktor entfernt. Nach 24 Stunden bei 0° C lässt die Chlorwasserstoffentwicklung nach. Der überschussige Fluorwasserstoff wird abdestilliert, der Rückstsnd in 1,5 l Methylenchlorid gelöst und mit Wasser extrahiert. Durch destillative Trennung der organischen Phase erhält man 175,5 g (71,3 % d.Th.) 2-Perfluoräthoxy-chlorbenzol, Sdp. 154° C, $n_D^{25}$ = 1,4106.

Beispiel 5: 4-Perfluoräthoxy-chlorbenzol.

In einem 500 ml Polytetrafluoräthylen-Reaktor, der mit Rührwerk, Thermometer und Rückflusskühler ausgerüstet ist, werden 52,6 g (0,2 Mol) 4-(1-Chlor-1,2,2,2-tetrafluoräthoxy)-chlorbenzol in 100 ml Fluorwasserstoff vorgelegt. Bei einer Temperatur zwischen -5° C und 0° C werden 6,0 g (0,02 Mol; entsprechend 10 Mol % Antimonpentachlorid) zugegeben. Der entstehende Chlorwasserstoff wird über den Rückflusskühler aus dem Reaktor entfernt. Nach 4 Stunden bei einer Temperatur zwischen 0° C und +5° C lässt die Chlorwasserstoffentwicklung nach. Der überschüssige Fluorwasserstoff wird abdestilliert, der Rückstand in 200 ml Methylenchlorid gelöst und mit Wasser extrahiert. Durch destillative Trennung der organischen Phase erhält man 43,7 g (89,5 % d.Th.) 4-Perfluoräthoxy-chlorbenzol, Sdp. 151° C.

Beispiel 6: 4-Perfluoräthoxy-chlorbenzol.

In einem 500 ml Polytetrafluoräthylen-Reaktor, der mit Rührwerk, Thermometer und Rückflusskühler ausgerüstet ist, werden 55,9 g (0,2 Mol) 4-(1,1-Dichlor-2,2,2-trifluoräthoxy)-chlorbenzol in 16 g Fluorwasserstoff vorgelegt. Bei einer Temperatur von +10° C werden 6,0 g (0,02 Mol, entsprechend 10 Mol-%) Antimonpentachlorid zugegeben. Der entstehende Chlorwasserstoff wird über den Rückflusskühler aus dem Reaktor entfernt. Nach 1,5 Stunden bei +10° C läßt die Chlorwasserstoffentwicklung nach. Der Rückstand wird in 200 ml Methylenchlorid gelöst und mit Wasser extrahiert. Durch destillative Trennung der organischen Phase erhält man 44,7 g (90,4 % d.Th.) 4-Perfluoräthoxy-chlorbenzol, Sdp. 151° C.

Beispiel 7: 4-(1-Chlor-1,2,2,2-tetrafluoräthoxy)-chlorbenzol.

In einem 500 ml Polytetrafluoräthylen-Reaktor, der mit Rührwerk, Thermometer und Rückflusskühler ausgerüstet ist, werden 55,9 g (0,2 Mol) 4-(1,1-Dichlor-2,2,2-trifluoräthoxy)-chlorbenzol in 100 ml Fluorwasserstoff vorgelegt. Bei einer Temperatur zwischen -5° C und 0° C werden 3,0 g (0,01 Mol; entsprechend 5 Mol-%) Antimonpentachlorid zugegeben. Der entstehende Chlorwasserstoff wird über den Rückflusskühler aus dem Reaktor entfernt. Nach 20 Minuten bei 0°C läßt die Chlorwasserstoffentwicklung nach. Der überschüssige Fluorwasserstoff wird abdestilliert, der Rückstand in 200 ml Methylenchlorid gelöst und mit Wasser extrahiert. Durch destillative Trennung der organischen Phase erhält man 47,3 g (89,8 % d.Th.) 4-(1-Chlor-1,2,2,2-tetrafluoräthoxy)-chlorbenzol, Sdp. 181° C, $n_D^{25}$ = 1,4362.

Beispiel 8: 4-Perfluoräthoxy-fluorbenzol

In einem 500 ml Polytetrafluoräthylen-Reaktor, der mit Rührwerk, Thermometer und Rückflusskühler ausgerüstet ist, werden 17,0 g (0,0646 Mol) 4-(1,1-Dichlor-2,2,2-trifluoräthoxy)-fluorbenzol und 35 g Fluorwasserstoff vorgelegt. Bei einer Temperatur von +10° C werden 2,0 g ($6,7 \cdot 10^{-3}$ Mol, entsprechend 10 Mol-%) Antimonpentachlorid zugegeben. Der entstehende Chlorwasserstoff wird über den Rückflusskühler aus dem Reaktor entfernt. Nach 1 Stunde wird der Reaktionsmischung nochmals 1,0 g Antimonpentachlorid zugesetzt. Nach insgesamt 4 Stunden Reaktionsdauer wird der Fluorwasserstoff abdestilliert, der Rückstand in 150 ml Methylenchlorid gelöst und mit Wasser extrahiert. Durch destillative Trennung der organischen Lösung erhält man 4-Perfluoräthoxy-fluorbenzol, Sdp: 121° C, $n_D^{26}$ = 1,3710.

Beispiel 9: 1-Chlor-1,2,2,2-tetrafluoräthylthio-benzol.

In einem 500 ml polytetrafluoräthylen-Reaktor, der mit Rührwerk, Thermometer und Rückflusskühler ausgerüstet ist, werden 24,7 g (0,094 Mol) 1,1-Dichlor-2,2,2-trifluoräthylthio-benzol vorgelegt und anschliessend 50 g Fluorwasserstoff einkondensiert. Bei einer Temperatur von 20° C werden 4,2 g (0,014 Mol; entsprechend 15 Mol-%) Antimonpentachlorid zugegeben. Der entstehende Chlorwasserstoff wird über den Rückflusskühler aus dem Reaktor enfernt. Nach 3 Stunden werden nochmals 1,4 g (5 Mol-%), nach 4 Stunden 2,8 g (10 Mol-%) Antimonpentachlorid zugesetzt. Nach insgesamt 5 Stunden Reaktionsdauer wird der Fluorwasser abdestilliert, der Rückstand in 150 ml Methylenchlorid gelöst und mit Wasser extrahiert. Durch destillative Trennung der Lösung erhält man 1-Chlor-1,2,2,2-tetrafluoräthylthiobenzol, Sdp. 178 - 179° C, $n_D^{20}$ = 1,4732.

**Patentansprüche**

1. Verfahren zur Herstellung eines α,α-Difluoralkyl-phenyläther der Formel I

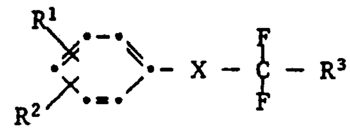 (I)

worin
$R^1$ Wasserstoff, Halogen, Amino, Nitro, $-SO_2-R^4$, $-S-R^5$, $-SO-R^6$ oder $-S-S-R^7$,
$R^2$ Wasserstoff, Halogen, Nitro, Hydroxyl oder $-SO_2-R^8$,
$R^3$ $C_1$-$C_5$-Halogenalkyl,
X Sauerstoff Schwefel, -SO- oder $-SO_2-$,
$R^4$ Hydroxyl, Halogen, Amino, -N=C=O, -NH-CO-Cl, -NH-CO-Br, $-NR^9R^{10}$, Benzyl, Phenyl, $C_1$-$C_4$-Alkyl oder $-NH-CO-NR^{11}R^{12}$,
$R^5$ und $R^6$ $C_1$-$C_4$-Alkyl, Phenyl oder Benzyl,
$R^7$ $C_1$-$C_4$-Alkyl, Phenyl oder Benzyl,
$R^8$ Hydroxyl oder Halogen,
$R^9$ und $R^{10}$ unabhängig voneinander $C_1$-$C_4$-Alkyl oder Benzyl und
$R^{11}$ und $R^{12}$ unabhängig voneinander Wasserstoff, $C_1$-$C_4$-Alkyl, Phenyl oder einen aromatischen Heterocyclus bedeuten,
dadurch gekennzeichnet, dass man eine Verbindung der Formel II

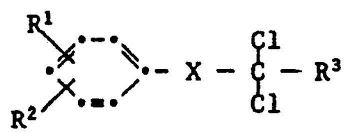 (II)

worin $R^1$, $R^2$, $R^3$ und X die unter Formel I gegebenen Bedeutungen haben, mit Fluorwasserstoff in Gegenwart einer katalytischen Menge eines Antimon(V)halogenids oder eines Antimontrichlorid/Halogengemisches fluoriert.

2. Verfahren gemäss Anspruch 1, dadurch gekennzeichnet, dass man als Fluorierungskatalysator Antimonpentachlorid verwendet.

3. Verfahren gemäss Anspruch 1, dadurch gekennzeichnet, dass man 0,1 bis 50 Mol-Prozent an Antimon(V)halogenid oder eines Antimontrichlorid/Halogengemisches, bezogen auf die eingesetzte Menge der Ausgangsverbindung der Formel II, verwendet.

4. Verfahren gemäss Anspruch 3, dadurch gekennzeichnet, dass man 1 bis 20 Mol-Prozent, vorzugsweise 5 bis 10 Mol-Prozent, an Antimon(V)halogenid verwendet.

5. Verfahren gemäss Anspruch 1, dadurch gekennzeichnet, dass man die Umsetzung bei einer Temperatur zwischen -20°C und +100°C ausführt.

6. Verfahren gemäss Anspruch 1, dadurch gekennzeichnet, dass man die Umsetzung bei einer Temperatur zwischen -10°C und +20°C ausführt.

7. Verfahren gemäss Anspruch 1, dadurch gekennzeichnet, dass man die Umsetzung mit einem Überschuss von Fluorwasserstoff durchführt.

8. Verfahren gemäss Anspruch 1, dadurch gekennzeichnet, dass man die Umsetzung bei einem Druck zwischen 0,1 bar und 20 bar, vorzugsweise bei Normaldruck, ausführt.

9. Verfahren gemäss Anspruch 1, dadurch gekennzeichnet, dass man eine Verbindung der Unterformel Ia

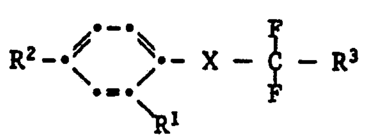 (Ia)

herstellt, worin
$R^1$ Wasserstoff, Halogen, Nitro, Amino, $-SO_2-R^4$, $-S-R^5$ oder $-SO-R^6$,
$R^2$ Wasserstoff Halogen oder Nitro,
$R^3$ $C_1$-$C_3$-Perfluoralkyl,
X Sauerstoff oder Schwefel,
$R^4$ Hydroxyl, Amino, Phenyl, Benzyl, $C_1$-$C_4$-Alkyl oder Halogen, vorzugsweise Fluor oder Chlor und
$R^5$ und $R^6$ $C_1$-$C_4$-Alkyl oder Benzyl bedeuten.

10. Verfahren gemäß Anspruch 9, dadurch gekennzeichnet, dass $R^1$ für $-SO_2-NH_2$, Wasserstoff oder Chlor, $R^2$ für Wasserstoff oder Chlor, $R^3$ für $C_1$-$C_3$-Perfluoralkyl und X für Sauerstoff oder Schwefel stehen.

11. Verfahren gemäß Anspruch 10, dadurch gekennzeichnet, dass eines von $R^1$ und $R^2$ für Chlor und das andere für Wasserstoff oder Chlor steht.

12. Verfahren gemäß Anspruch 1, dadurch gekennzeichnet, dass man die Verbindung 2-Perfluoräthoxyphenylsulfonamid herstellt.

13. Verfahren gemäß Anspruch 10, dadurch gekennzeichnet, dass man eine Verbindung aus der Gruppe 2,4-Dichlor-perfluoräthoxybenzol, 2-Perfluoräthoxy-chlorbenzol oder 4-Perfluoräthoxy-chlorbenzol herstellt.

14. Verfahren gemäß Anspruch 1, dadurch gekennzeichnet, dass man die Umsetzung in Gegenwart von 0,1 bis 50 Mol-Prozent an Antimon(V)halogenid bei einer Temperatur zwischen -20°C und +100°C unter einem Druck zwischen 0,1 bar und 20 bar ausführt.

15. Verfahren gemäß Anspruch 1, dadurch gekennzeichnet, dass man die Umsetzung in Gegenwart von 1 bis 20 Mol-Prozent an Antimon(V)chlorid bei einer Temperatur zwischen -10°C und +20°C unter Normaldruck in flüssigem Fluorwasserstoff ausführt.

16. Verfahren gemäß Anspruch 1 zur Herstellung einer Verbindung aus der Gruppe 2-Perfluoräthoxyphenylsulfonamid, 2,4-Dichlor-perfluor-äthoxybenzol, 2-Perfluoräthoxy-chlorbenzol oder 4-Perfluoräthoxychlorbenzol, dadurch gekennzeichnet, dass man 2-(1,1-Dichlor-2,2,2-trifluoräthoxy)-phenylsulfonamid, 1-(1,1-Dichlor-2,2,2-trifluoräthoxy)-2,4-dichlorbenzol, 2-(1,1-Dichlor-2,2,2-trifluoräthoxy)-chlorbenzol oder 4-(1,1-Dichlor-2,2,2-trifluoräthoxy)-chlorbenzol in Gegenwart von 5 bis 20 Mol-Prozent Antimon(V)chlorid bei einer Temperatur zwischen -10°C und +10°C unter Normaldruck in flüssigem Fluorwasserstoff fluoriert.

17. Verfahren gemäß Anspruch 1, dadurch gekennzeichnet, dass man das Verfahren in 2 Stufen ausführt, indem man zunächst die Verbindung der Formel II in Gegenwart von 0,1 bis 5 Mol-Prozent Antimon(V)halogenid mit Fluorwasserstoff in das Zwischenprodukt der Formel III

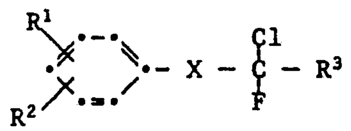 (III)

worin $R^1$, $R^2$, $R^3$ und X die unter Formel I im Anspruch 1 gegebenen Bedeutungen haben, überführt und dieses in Gegenwart von 1 bis 20 Mol-Prozent an Antimon(V)halogenid mit Fluorwasserstoff zur Verbindung der Formel I umsetzt, wobei die Umsetzung der Verbindung der Formel II zur Verbindung der Formel III bei einer Temperatur zwischen -20°C und 0°C und die weitere Umsetzung des Zwischenprodukts der Formel III bei einer Temperatur zwischen 0°C und +100°C durchgeführt wird.

18. Verbindungen der Formel IIIb

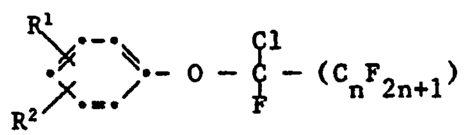 (IIIb),

worin $R^1$ und $R^2$ die im Anspruch 1 unter Formel I gegebenen Bedeutungen haben und n für eine Zahl von eins bis fünf steht.

19. Verbindungen der Formel IIIc

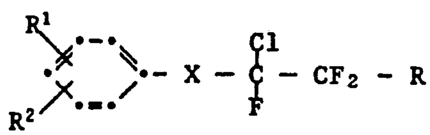 (IIIc),

worin $R^1$ und $R^2$ die im Anspruch 1 unter Formel I gegebenen Bedeutungen haben X für Schwefel, SO oder $SO_2$ und R für Fluor oder $C_1$-$C_4$-Perhalogenalkyl stehen.

**Claims**

1. A process for the preparation of an α,α-difluoroalkyl phenyl ether of formula I

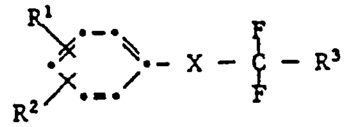 (I)

in which

$R^1$ is hydrogen, halogen, amino, nitro, $-SO_2-R^4$, $-S-R^5$, $-SO-R^6$ or $-S-S-R^7$,

$R^2$ is hydrogen, halogen, nitro, hydroxyl or $-SO_2-R^8$,

$R^3$ is $C_1$-$C_5$haloalkyl,
X is oxygen, sulfur, -SO- or $-SO_2-$,
$R^4$ is hydroxyl, halogen, amino, -N=C=O, -NH-CO-Cl, -NH-CO-Br, $-NR^9R^{10}$, benzyl, phenyl, $C_1$-$C_4$alkyl or $-NH-CO-NR^{11}R^{12}$,
$R^5$ and $R^6$ are $C_1$-$C_4$alkyl, phenyl or benzyl,
$R^7$ is $C_1$-$C_4$alkyl, phenyl or benzyl,
$R^8$ is hydroxyl or halogen,
$R^9$ and $R^{10}$ independently of one another are $C_1$-$C_4$alkyl or benzyl and
$R^{11}$ and $R^{12}$ independently of one another are hydrogen, $C_1$-$C_4$alkyl, phenyl or an aromatic heterocycle,
which process comprises fluorinating a compound of formula II

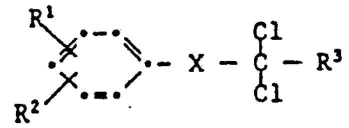 (II)

wherein $R^1$, $R^2$, $R^3$ and X are as defined for formula I, with hydrogen fluoride, in the presence of a catalytic amount of an antimony(V) halide or of an antimony trichloride/halogen mixture.

2. A process according to claim 1, which comprises the use of antimony pentachloride as the fluorination catalyst.

3. A process according to claim 1, which comprises the use of 0.1 to 50 mol per cent of antimony(V)halide or of an antimony trichloride/halogen mixture, based on the amount of the starting material of formula II employed.

4. A process according to claim 3, which comprises the use of 1 to 20 mol per cent, preferably 5 to 10 mol per cent, of antimony(V)halide.

5. A process according to claim 1, which comprises carrying out the reaction at a temperature between -20°C and +100°C.

6. A process according to claim 1, which comprises carrying out the reaction at a temperature between -10°C and +20°C.

7. A process according to claim 1, which comprises carrying out the reaction with an excess of hydrogen fluoride.

8. A process according to claim 1, which comprises carrying out the reaction under a pressure between 0.1 and 20 bar, preferably under normal pressure.

9. A process according to claim 1, which comprises preparing a compound of sub-formula Ia

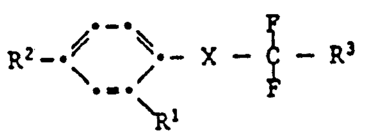 (Ia)

in which
$R^1$ is hydrogen, halogen, nitro, amino, $-SO_2-R^4$, $-S-R^5$ or $-SO-R^6$,
$R^2$ is hydrogen, halogen or nitro,
$R^3$ is $C_1$-$C_3$perfluoroalkyl,
X is oxygen or sulfur,
$R^4$ is hydroxyl, amino, phenyl, benzyl, $C_1$-$C_4$alkyl or halogen, preferably fluorine or chlorine and
$R^5$ and $R^6$ are $C_1$-$C_4$alkyl or benzyl.

10. A process according to claim 9, wherein $R^1$ is $-SO_2-NH_2$, hydrogen or chlorine, $R^2$ is hydrogen or chlorine, $R^3$ is $C_1$-$C_3$perfluoroalkyl and X is oxygen or sulfur.

11. A process according to claim 10, wherein one of $R^1$ and $R^2$ is chlorine and the other is hydrogen or chlorine.

12. A process according to claim 1, which comprises preparing the compound 2-perfluoroethoxyphenylsulfonamide.

13. A process according to claim 10, which comprises preparing a compound from the group consisting of 2,4-dichloroperfluoroethoxybenzene, 2-perfluoroethoxychlorobenzene or 4-perfluoroethoxychlorobenzene.

14. A process according to claim 1, which comprises carrying out the reaction in the presence of 0.1 to 50 mol per cent of antimony(V)halide, at a temperature between -20°C and +100°C and under a pressure between 0.1 and 20 bar.

15. A process according to claim 1, which comprises carrying out the reaction in the presence of 1 to 20 mol per cent of antimony(V)chloride, at a temperature between -10°C and +20°C and under normal pressure, in liquid hydrogen fluoride.

16. A process according to claim 1 for the preparation of a compound from the group consisting of 2-perfluoroethoxyphenylsulfonamide, 2,4-dichloroperfluoroethoxybenzene, 2-perfluoroethoxychlorobenzene or 4-perfluoroethoxychlorobenzene, which process comprises fluorinating 2-(1,1-dichloro-2,2,2-trifluoroethoxy)phenylsulfonamide, 1-(1,1-dichloro-2,2,2-trifluoroethoxy)-2,4-dichlorobenzene, 2-(1,1-dichloro -2,2,2-trifluoroethoxy)chlorobenzene or 4-(1,1-dichloro-2,2,2-trifluoro-ethoxy)chlorobenzene, in the presence of 5 to 20 mol per cent of antimony(V)chloride, at a temperature between -10°C and +10°C and under normal pressure, in liquid hydrogen fluoride.

17. A process according to claim 1, which comprises carrying out the process in 2 steps, by first converting the compound of formula II with hydrogen fluoride into the intermediate of the formula III

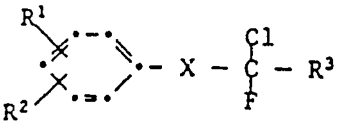

(III)

wherein $R^1$, $R^2$, $R^3$ and X are as defined for formula I in claim 1, in the presence of 0.1 to 5 mol per cent of antimony(V)halide, and reacting said intermediate with hydrogen fluoride, in the presence of 1 to 20 mol per cent of antimony(V) halide, to give the compound of formula I, the reaction of the compound of formula II to give the compound of formula III being carried out at a temperature between -20°C and 0°C and the further reaction of the intermediate of formula III being carried out at a temperature between 0°C and +100°C.

18. A compound of formula IIIb

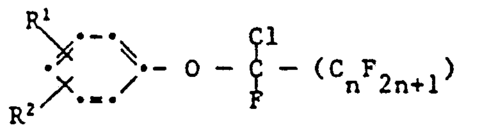

(IIIb),

in which $R^1$ and $R^2$ are as defined for formula I in claim 1 and n is a number from one to five.

19. A compound of formula IIIc

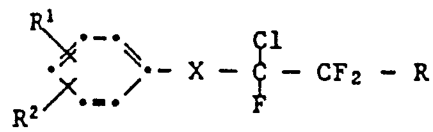

(IIIc),

in which $R^1$ and $R^2$ are as defined for formula I in claim 1, X is sulfur, SO or $SO_2$ and R is fluorine or $C_1$-$C_4$perhaloalkyl.

**Revendications**

1. Procédé pour préparer un oxyde d'α,α-difluoralkyle et de phényle répondant à la formule I:

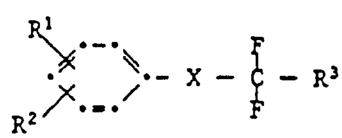

(I)

dans laquelle:

$R^1$ représente l'hydrogène, un halogène ou un radical amino, nitro, $-SO_2-R^4$, $-S-R^5$, $-SO-R^6$ ou $-S-S-R^7$,

$R^2$ représente l'hydrogène, un halogène ou un radical nitro, hydroxy ou $-SO_2-R^8$,

$R^3$ représente un radical halogéno-alkyle en $C_1$-$C_5$,

X représente l'oxygène, le soufre ou un radical -SO- ou $-SO_2-$,

$R^4$ représente un radical hydroxy, un halogène ou un radical amino, -N=C=O, -NH-CO-Cl, -NH-CO-Br, $-NR^9R^{10}$, benzyle, phényle, alkyle en $C_1$-$C_4$ ou $-NH-CO-NR^{11}R^{12}$,

$R^5$ et $R^6$ représentent chacun un alkyle en $C_1$-$C_4$, un phényle ou un benzyle,

$R^7$ représente un alkyle en $C_1$-$C_4$ un phényle ou un benzyle,

$R^8$ représente un hydroxy ou un halogène,

$R^9$ et $R^{10}$ représentent chacun, indépendamment l'un de l'autre, un alkyle en $C_1$-$C_4$ ou un benzyle et

$R^{11}$ et $R^{12}$ représentent chacun, indépendamment l'un de l'autre, l'hydrogène, un alkyle en $C_1$-$C_4$ un phényle ou un hétérocycle aromatique,

procédé caractérisé en ce qu'on fluore un composé répondant à la formule II:

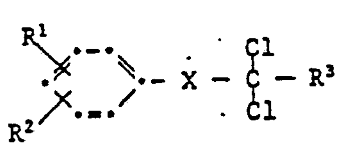

(II)

dans laquelle $R^1$, $R^2$, $R^3$ et X ont les significations données au-dessous de la formule I, au moyen du fluorure d'hydrogène, en présence d'une quantité catalytique d'un halogénure d'antimoine(V) ou d'un mélange de trichlorure d'antimoine et d'un halogène.

2. Procédé selon la revendication 1 caractérisé en ce qu'on utilise le pentachlorure d'antimoine comme catalyseur de fluoration.

3. Procédé selon la revendication 1 caractérisé en ce qu'on utilise de 0,1 à 50 % en moles d'un halogénure d'antimoine(V) ou d'un mélange de trichlorure d'antimoine et d'un halogène, par rapport à la quantité mise en jeu du corps de départ de formule II.

4. Procédé selon la revendication 3 caractérisé en ce qu'on utilise de 1 à 20 % en moles d'un halogénure d'antimoine(V), de préférence de 5 à 10 % en moles.

5. Procédé selon la revendication 1 caractérisé en ce qu'on effectue la réaction à une température comprise entre -20 et +100° C.

6. Procédé selon la revendication 1 caractérisé en ce qu'on effectue la réaction à une température comprise entre -10 et +20° C.

7. Procédé selon la revendication 1 caractérisé en ce qu'on effectue la réaction avec un excès de fluorure d'hydrogène.

8. Procédé selon la revendication 1 caractérisé en ce qu'on effectue la réaction sous une pression comprise entre 0,1 et 20 bar, de préférence sous la pression normale.

9. Procédé selon la revendication 1 caractérisé en ce qu'on prépare un composé répondant à la sous-formule Ia:

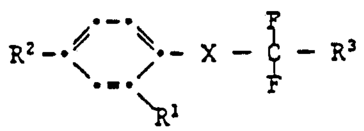

(Ia)

dans laquelle

$R^1$ représente l'hydrogène, un halogène ou un radical nitro, amino, $-SO_2-R^4$, $-S-R^5$ ou $-SO-R^6$,

$R^2$ représente l'hydrogène, un halogène ou un radical nitro,

$R^3$ représente un radical perfluoroalkyle en $C_1$-$C_3$,

X représente l'oxygène ou le soufre,

$R^4$ représente un hydroxy, un amino, un phényle, un benzyle, un alkyle en $C_1$-$C_4$ ou un halogène, de préférence le fluor ou le chlore, et

$R^5$ et $R^6$ représentent chacun un alkyle en $C_1$-$C_4$ ou un benzyle.

10. Procédé selon la revendication 9 caractérisé en ce que $R^1$ représente un radical $-SO_2-NH_2$, l'hydrogène ou le chlore, $R^2$ représente l'hydrogène ou le chlore, $R^3$ représente un radical perfluoralkyle en $C_1$-$C_3$ et X représente l'oxygène ou le soufre.

11. Procédé selon la revendication 10 caractérisé en ce que l'un des symboles $R^1$ et $R^2$ représente le chlore et l'autre l'hydrogène ou le chlore.

12. Procédé selon la revendication 1 caractérisé en ce qu'on prépare le perfluoréthoxy-2 benzène-sulfonamide.

13. Procédé selon la revendication 10 caractérisé en ce qu'on prépare un composé de l'ensemble comprenant le (dichloro-2,4 perfluoréthoxy)-benzène, le perfluoréthoxy-2 chlorobenzène et le perfluoréthoxy-4 chlorobenzène.

14. Procédé selon la revendication 1 caractérisé en ce qu'on effectue la réaction en présence de 0,1 à 50 % en moles d'un halogénure d'antimoine(V), à une température comprise entre -20 et +100°C et sous une pression comprise entre 0,1 et 20 bar.

15. Procédé selon la revendication 1 caractérisé en ce qu'on effectue la réaction en présence de 1 à 20 % en moles de chlorure d'antimoine(V), à une température comprise entre -10 et +20°C, sous la pression normale, dans du fluorure d'hydrogène liquide.

16. Procédé selon la revendication 1 pour la préparation d'un composé de l'ensemble comprenant le perfluoréthoxy-2 benzène-sulfonamide, le (dichloro-2,4-perfluoréthoxy)-benzène, le perfluoréthoxy-2 chlorobenzène et le perfluoréthoxy-4 chlorobenzène, procédé caractérisé en ce qu'on fluore le (dichloro-1,1 trifluoro-2,2,2 éthoxy)-2 benzène-sulfonamide, le (dichloro-1,1 trifluoro-2,2,2 éthoxy)-1 dichloro-2,4 benzène, le (dichloro-1,1 trifluoro-2,2,2 éthoxy)-2 chlorobenzène ou le (dichloro-1,1 trifluoro-2,2,2 éthoxy)-4 chlorobenzène en présence de 5 à 20 % en moles de chlorure d'antimoine(V), à une température comprise entre -10 et +10° C, sous la pression normale, dans du fluorure d'hydrogène liquide.

17. Procédé selon la revendication 1 caractérisé en ce qu'on effectue le procédé en deux étapes, la première consistant à transformer le composé de formule II en présence de 0,1 à 5 % en moles d'un halogénure d'antimoine(V), au moyen du fluorure d'hydrogène, en le corps intermédiaire répondant à la formule III:

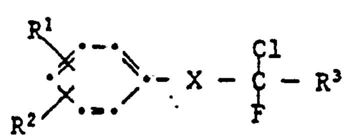

(III)

dans laquelle $R^1$, $R^2$, $R^3$ et X ont les significations qui leur ont été données à la revendication 1 à propos de la formule I, et la deuxième consistant à faire réagir ce corps intermédiaire, en présence de 1 à 20 % en moles d'un halogénure d'antimoine(V), avec le fluorure d'hydrogène pour le convertir en le composé de formule I, la transformation du composé de formule II en composé de formule III étant effectuée à une température comprise entre -20 et 0° C et la réaction ultérieure du corps intermédiaire de formule III étant effectuée à une température comprise entre 0 et +100° C.

18. Composés répondant à la formule IIIb:

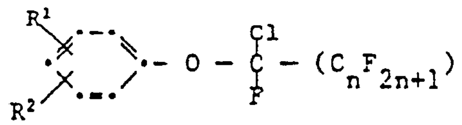

(IIIb)

dans laquelle $R^1$ et $R^2$ ont les significations qui leur ont été données à la revendication 1 à propos de la formule I, et n désigne un nombre de 1 à 5.

19. Composés qui répondent à la formule IIIc:

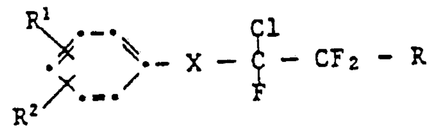

(IIIc)

dans laquelle $R^1$ et $R^2$ ont les significations qui leur ont été données à la revendication 1 à propos de la formule I, X représente le soufre, un radical SO ou un radical $SO_2$, et R représente le fluor ou un radical perhalogéno-alkyle en $C_1$-$C_4$.